# EUROPEAN PATENT APPLICATION

(11) **EP 1 719 523 A1**
(43) Date of publication of application: **08.11.2006**
(21) Application number: 05704198.0
(22) Date of filing: 27.01.2005
(51) Int. Cl.: A61K 38/27, A61K 38/28, A61K 38/21, A61K 47/02, A61K 47/34, A61K 9/08, A61K 9/52

(54) **PROTEIN DRUG SUSTAINED-RELEASE MICROPARTICLE PREPARATION FOR INJECTION AND PROCESS FOR PRODUCING THE SAME**

(30) Priority: 26.02.2004 JP 2004051526
(71) Applicant: Japan Science and Technology Agency, Kawaguchi-shi, Saitama 332-0012 (JP); Galenisearch Laboratories, Kawasaki-shi, Kanagawa 2100855 (JP)
(72) Inventor: OGAWA, Yasuaki, Otokuni-gun, Kyoto 6180071 (JP); MIYAMOTO, Yoko, Kanagawa 2470072 (JP); NIIMI, Jun, Kawasaki-shi, Kanagawa 2120055 (JP); FUJII, Takao, Koutou-ku, Tokyo 1350051 (JP)
(74) Representative: Rupp, Christian
(86) International application number: PCT/JP2005/001095
(87) International publication number: WO 2005/082405

(57) **Abstract**

To provide protein drug sustained-release microparticle preparations for injection that in the production thereof, minimize the use of organic solvents and avoid the simultaneous use of an organic solvent immiscible with water and an aqueous solution and that with respect to the obtained product, simultaneously have in vivo disappearing and sustained-release capabilities, slowly release the contained protein drug at a substantially constant rate over a period of three days or more and realize a drug content of 5% or more, excelling in dispersibility and needle passability; and to provide a process for producing the same. The protein drug sustained-release microparticle preparations for injection comprise a porous apatite or derivative thereof containing a protein drug and, provided thereon by coating or adhesion, an in vivo disappearing polymer.

## Description

### Technical Field

The present invention relates to protein drug sustained-release microparticle preparations for injection comprising, as a base, microparticles of a porous apatite or derivative thereof that slowly disappear in vivo, and to a process for producing the same.

### Background Art

Investigation has heretofore been made on protein drug sustained-release preparations for injection that slowly release the protein drugs over a long period, most of which comprise copolylactic-glycolic acid (PLGA) as abase (see e.g., Patent Documents 1 and 2 and Non-Patent Documents 1, 2, and 3). Actually, sustained-release microcapsules that contain human growth hormone (hGH) and comprise PLGA as a base are in practical use in treatment in U.S. (see e.g., Non-Patent Document 4). PLGA is a biodegradable base that hydrolyzes and slowly disappears in a living body , and this property is preferable for a base of an injection. To produce sustained-release preparations using PLGA, organic solvents for dissolving it are generally used. On the other hand, many protein drugs are water-soluble and are therefore used together with an organic solvent solution and an aqueous solution in order to produce their sustained-release microparticle preparations using PLGA.

The simultaneous use of these two solvents leads to the denaturation and deactivation of some protein drugs. Such reduction in activity not only impairs efficacy but poses the risk of adversely affecting a living body. Water-soluble protein drug sustained-release microparticle preparations inevitably invite the transient excessive release of the protein drug in the early stage of administration (immediately after administration). Human growth hormone (protein drug) sustained-release microparticle preparations for injection using hydroxyapatite have also been reported (see e.g., Non-Patent Documents 5 and 6). However, all the preparations are two-component systems that have a particle size of apatite as large as 40 to 80 µm or 200 µm and are therefore difficult to administer by injection with a thin needle. Furthermore, their in vivo sustained-release effects are unknown. Besides, the amount of human growth hormone contained in apatite was as low as 1% or less.

Patent Document 1: Japanese Patent Laid-Open No. 10-231252
Patent Document 2: U.S. Patent No. 5656297
Non-Patent Document 1 : O.L. Johnson et al : Nature Medicine, 2: 795-799, (1996)
Non-Patent Document 2: M. Takenaga et al: J. Pharmacy Pharmacology, 54: 1189-1194, (2002)
Non-Patent Document 3: S. Takada et al: J. Controlled Release, 88: 229-242, (2003)
Non-Patent Document 4: NDA 21-075
Non-Patent Document 5: J. Guicheux et al: J. Biomedical Materials Research, 34: 165-170, (1997)
Non-Patent Document 6: H. Gautier et al: J. Biomedical Materials Research, 40: 606-613, (1998)

### Disclosure of the Invention

For protein drug sustained-release preparations for injection, there are many challenges to produce the preparations: materials having in vivo disappearing capabilities, which disappear from living bodies toward the end of drug release after administration, must be selected; in the production thereof, the preparations must avoid the simultaneous use of an organic solvent immiscible with water and an aqueous solution and circumvent the denaturation of the protein drugs; moreover, a drug content in the microparticle preparation must be at least 5% or more, otherwise it is difficult to administer with a thin needle due to their too large doses; the preparations must be able to pass through a thin needle because they are repetitively administered in many cases; and the microparticle preparations must slowly release the contained protein drug over a period of at least three days or more, preferably one week or more; and must minimize initial burst release likely to cause side effects.

Thus, an object of the present invention is to provide protein drug sustained-release microparticle preparations for injection that in the production thereof, minimize the use of organic solvents and avoid the simultaneous use of an organic solvent immiscible with water and an aqueous solution and that with respect to the resulting product, have both bioerodibilities and sustained-release capabilities, slowly release the contained protein drug at a substantially constant rate over a period of three days or more and realize a drug content of 5% or more, excelling in dispersibility and needle passability, and to provide a process for producing the same.

To solve these challenges, the present inventors have found that preparations that have both bioerodibilities and sustained-release capabilities are obtained by utilizing microparticles of a porous apatite or derivative thereof without the simultaneous use of water and an organic solvent. The present inventors have further found that the initial burst release of a protein drug is suppressed by utilizing the protein drug in combination with a water-soluble divalent metal compound. In addition, the present inventors have also found that sustained-release capabilities over a longer period and smaller initial burst release are simultaneously attained by sufficiently infiltrating a protein drug into a porous apatite and providing an in vivo disappearing polymer compound thereon by coating or adhesion.

The porous apatite and derivative thereof constituting the protein drug sustained-release microparticle preparation for injection described herein may be hydroxyapatite or a compound in which a portion of calcium as a component thereof is substituted with zinc. In this context, the rate of zinc substitution is preferably 1 to 20%. Microparticles of the porous apatite and derivative thereof can be obtained by a known method. Examples of the method include the method described in T. Yamaguchi, H. Yanagida (eds.), A. Makishima, H. Aoki, Ceramic Science Series 7: Bioceramics, GIHODO SHUPPAN Co., Ltd., pp. 7-9, 1984. In vivo disappearing speed differs depending on the ratio of calcium (Ca) and phosphorus (P) constituting hydroxyapatite. If a value of (Ca+Zn)/P is smaller than 1.67, higher water-solubility and higher in vivo disappearing speed are attained. It is preferred that the value of (Ca+Zn)/P should fall within the range of 1.67 to 1.51. A lower treatment temperature at which the apatite is produced renders water solubility higher and disappearing speed higher. The treatment temperature used is generally room temperature to 800°C, preferably 150°C to 600°C, more preferably 150°C to 400°C. If the apatite is burned at 800°C or higher, it does not disappear in vivo. If the apatite is treated at 100°C or lower, particles thereof tend to agglomerate together and are therefore difficult to administer by injection with an ordinary needle. The apatite is preferably used at a particle size of 50 µm or lower in average. However, if the particle size is too small, the encapsulation rate of the protein drug might be decreased. Therefore, the particle size used is preferably 0.1 to 50 µm, more preferably 0.5 to 30 µm, even more preferably 0.5 to 10 µm.

The in vivo disappearing polymer compound for coating this porous apatite includes polylactic acid (PLA) or copolylactic-glycolic-acid (PLGA), a block copolymer comprising PLA and/or PLGA bound with polyethylene glycol (PEG), collagen, polycyanoacrylic acid, and polyamino acid derivatives. PLA or PLGA used at a high concentration allows apatite particles coated with the in vivo disappearing polymer to agglomerate together. Since an organic solvent immiscible with water is used in this procedure, the protein drug might be denatured due to water added in freeze drying at the final step unless this organic solvent is completely removed. From studies in various ways, it has been concluded that the block copolymer comprising PLA or PLGA bound with PEG is preferable. This block copolymer may be a compound with a binding style in which PLA or PLGA is bound through ester bond with hydroxyl groups at both ends of PEG or bound through ester bond with hydroxyl group at one end of PEG. For the ester bond with one end of PEG, it is preferred that the other end should be protected with OCH₃, an alkoxy group, or the like. Alternatively, it may be bound with a functional group such as amino and carboxyl groups. Concerning the ratio of PEG and PLA or PLGA, the block copolymer has preferably 20 to 90% by weight, more preferably 25 to 65% by weight, of PEG. The molecular weight of the block copolymer is preferably 3,000 to 20,000, more preferably 5,000 to 12,000, in its entirety. The amount of the biodegradable block copolymer used is in the range of generally 3 to 100% by weight, preferably 5 to 30%, with respect to that of the apatite derivative.

The water-soluble divalent metal compound includes zinc chloride, zinc acetate, zinc carbonate, calcium chloride, calcium hydroxide, ferrous or ferric chloride, ferrous or ferric hydroxide, and cobaltous or cobaltic chloride. The zinc chloride is particularly preferably used. The zinc chloride may be used in combination with sodium carbonate or sodium bicarbonate. The amount of the water-soluble divalent metal compound used differs depending on the encapsulated protein drug and is in the range of generally preferably 2 to 100% by weight, more preferably 2 to 30% by weight, with respect to that of the porous apatite.

The protein drug is defined as a compound having a molecular weight of 5,000 or more. Examples thereof include human growth hormone, hepatocyte growth factor (HGF), fibroblast growth factor (FGF), IGF-1, EGF, NK4, VEGF, NGF, BDNF, BMP, adiponectin, interferons (INF-α), interleukins (such as IL-2, IL-4, and 1L-5), EPO, G-CSF, insulin, ANP, TNF-α, and antibodies. The amount of the protein drug used differs depending on the protein drug activity and a sustained-release period. A larger amount of the protein drug encapsulated in the porous apatite is more preferable. The amount of the protein drug stably encapsulated in the porous apatite is generally 5 to 50% with respect to that of the apatite.

A process for producing the protein drug sustained-release microparticle preparation for injection of the present invention is generally performed by procedures as described below. Microparticles of a porous apatite or derivative thereof are dispersed in an aqueous solution of a protein drug, and the dispersion is stirred to sufficiently infiltrate the aqueous solution into the apatite. Then, the aqueous solution that could not be infiltrated therein is removed by centrifugation and so on. If necessary, an aqueous solution of a water-soluble divalent metal compound is further added thereto and stirred to infiltrate the aqueous solution thereinto. Subsequent filtration and vacuum drying or freeze drying give a powder containing the protein drug. This powder is dispersed in an aqueous solution or suspension of an biodegradable block copolymer or in an aqueous solution or suspension of a biodegradable block copolymer containing a solvent miscible with water (e.g., acetone and ethanol), the dispersion is stirred and, if necessary, supplemented with a stabilizer or the like, followed by freeze drying or vacuum drying to produce the preparation in a powder form. This powder, when actually administered, is dispersed in an appropriate dispersion medium and subcutaneously or intramuscularly administered by injection. The particle size of the finally obtained sustained-release microparticle preparation may be a size that allows the preparation to pass through an injection needle used in typical administration. In reality, the smaller size an injection needle has, the less a patient is scared. It is more preferred that the preparation should pass through an injection needle with a thickness of 25G or smaller defined by the international standard that specifies the thickness of an injection needle. The particle size of the sustained-release microparticle preparation that satisfies these requirements is 0.5 to 50 µm. Moreover, the sustained-release period of the protein drug differs depending on drug activity and so on and is generally preferably one week or more.

It was found that microparticle preparations obtained by the present invention slowly release the contained protein drug over a period of at least three days or more and realize quite small initial burst release and a protein drug content reaching even 30% at maximum. Moreover, the resulting preparations passed through a 25G injection needle. Furthermore, the microparticle preparations can be prepared finally into a powdered form by freeze drying, and the encapsulated protein drug is very stable.

### Examples

Hereinafter, the sustained-release and in vivo disappearing capabilities of preparations of the present invention will be illustrated with reference to Examples. However, the present invention is not intended to be limited to these Examples.

### (Example 1)

Two types of hydroxyapatites with zinc substitution (average particle size: 8 µm) burned at different temperatures were used to conduct a confirmation test on in vivo disappearance. Five SD male rats (6 weeks old) per group were used. The product treated at 180°C and the product treated at 400°C were separately dispersed in suspensions (0.1% Tween 80, 0.5% CMC-Na, and 5% mannitol aqueous solution), and 5 mg each of the products was bilaterally administered at a dose of 0.2 ml/rat to the middle part of the dorsal hypodermis. Residuals in the administration sites were excised periodically (on 3 hours, 1, 5, 10, 15, and 20 days) after administration. The wet weights and calcium levels thereof were measured (Tables 1 and 2). The wets weight were nearly doubled due to swelling and so on from 1 to 5 days after administration, while the calcium levels were slightly increased. Thereafter, the disappearance of both the wets weight and the calcium levels rapidly proceeded. They almost disappeared on around 15 days for the product treated at 180°C and on 20 days for the product treated at 400°C.

**Table 1 Wet weights of extracted hydroxyapatite (mg)**

| | 3 hours | 1 day | 5 days | 10 days | 15 days | 20 days |
|---|---|---|---|---|---|---|
| Product treated at 180°C (n=5) | 14.2 | 27.1 | 28.0 | 17.0 | 2.3 | 0.0 |
| Product treated at 400°C (n=5) | 14.0 | 23.9 | 29.1 | 23.2 | 10.8 | 0.0 |

**Table 2 Residual calcium levels of excised hydroxyapatite (mg)**

| | 3 hours | 1 day | 5 days | 10 days | 15 days | 20 days |
|---|---|---|---|---|---|---|
| Product treated at 180°C (n=5) | 1.27 | 1.53 | 1.45 | 0.26 | 0.01 | 0.00 |
| Product treated at 400°C (n=5) | 1.33 | 1.62 | 1.91 | 0.48 | 0.20 | 0.00 |

### (Example 2)

A derivative (HAp-Zn-0.5 (100 mg): 5% of calcium in hydroxyapatite (HAp) was substituted with zinc; 0.5 mol zinc with respect to 9.5 mol calcium) burned at 400°C was supplemented with 700 µL of hGH solution (50 mg/mL) desalted with a PD-10 column (Amersham Pharmacia) and then with water to bring the final amount of the solution to 5 mL. After stirring for 3 minutes, the solution was centrifuged at 3, 000 rpm for 3 minutes. The resulting pellet was supplemented with 10 mL of water and stirred for 1 minute. The suspension was centrifuged again at 3, 000 rpm for 3 minutes. The resulting pellet was supplemented with 2.0 mL of aqueous solution of 20.4 mg/mL zinc chloride (300 µmol zinc chloride; Wako Pure Chemical Industries, Ltd., Osaka, Japan) and stirred with a touch mixer, followed by freeze drying. PLA-PEG-PLA-Y004 (PEG ratio: 32%; molecular weight: 8,200) was dissolved at a concentration of 20% in acetone. This acetone solution was mixed with water at a 1:4 ratio to produce an acetone-water mixture solution containing the the block copolymer. The resulting freeze-dried powder was supplemented with 500 µL of this acetone-water mixture solution containing the polymer and well stirred with a touch mixer, followed by freeze drying. A preparation untreated with polymer solution was produced as a control. A hGH content in the resulting preparations was quantified with micro BCA protein assay kit (Pierce).

The in vitro release capabilities of the resulting hGH microparticle preparation samples were compared. The precisely weighed 2.5 mg aliquot of each of the resulting hGH microparticle preparation samples was supplemented with 0.250 mL of PBS (phosphate buffered saline) and stirred at 37°C. The supernatant was collected periodically by centrifugation at 3000 rpm for 3 min. The amount of hGH released into the supernatant was quantified by gel filtration HPLC analysis (TOSO G2000SW-xl). This result is shown in Table 3. The release of hGH into PBS was suppressed more in the preparation treated with polymer solution than in the preparation untreated with polymer solution produced as a control.

**Table 3 Influence of polymer solution treatment on in vitro release capabilities of hGH microparticle preparation**

| Polymer solution treatment | Cumulative amount of hGH released (µg) | | | | |
|---|---|---|---|---|---|
| | 2 hr | 4 hr | 24 hr | 4 day | 7 day |
| Untreated (n=2) | 0.8 | 2.3 | 6.3 | 7.8 | 9.6 |
| Treated (n=2) | 1.5 | 2.6 | 2.6 | 2.6 | 2.9 |

### (Example 3)

HAp-Zn-0.5 (100 mg) treated at 400°C was supplemented with 700 µL of hGH solution (50 mg/mL) desalted with a PD-10 column (Amersham Pharmacia) and then stirred for 1 minute with a touch mixer. Next, the solution was supplemented with water to bring the final amount of the solution to 5 mL, followed by stirring for 1 minute with a touch mixer. The suspension was left undisturbed for 3 minutes and centrifuged at 3,000 rpm for 3 minutes. The resulting pellet was supplemented with 5 mL of water and stirred again for 1 minute. The suspension was centrifuged again at 3, 000 rpm for 3 minutes. The resulting pellet was supplemented with 2.7 mL of aqueous solution of 20.4 mg/mL zinc chloride (400 µmol zinc chloride; Wako Pure Chemical Industries, Ltd., Osaka, Japan) and stirred with a touch mixer, followed by freeze drying.
PLA-PEG-PLA-Y001 (PEG ratio: 65.4%; molecular weight: 14,600) was dissolved at a concentration of 20% in acetone. This acetone solution was mixed with water at a 1:4 ratio to produce an acetone-water mixture solution containing the polymer. The resulting freeze-dried powder was supplemented with 500 µL of this acetone-water mixture solution containing the polymer and well stirred with a touch mixer, followed by freeze drying. A preparation untreated with polymer solution was produced as a control. A hGH content in the resulting hGH microparticle preparations was quantified with micro BCA protein assay kit (Pierce).

Each of the produced hGH microparticle preparations was suspended in 0.5% CMC-Na, 5% mannitol, and 0.1% Tween 80 and subcutaneously administered at 10 IU/kg (1 IU: 0.35 mg) to the dorsal site of a male SD rat.
Blood was collected from the tail vein at 1, 2, 4, and 8 hours after administration and subsequently on the daily basis to measure a hGH concentration in blood with an automatic EIA apparatus AIA-6000 (Tosoh) and E Test "TOSOH" II (HGH). This result is shown in Table 4. The higher hGH concentration in blood was maintained for a longer time in the preparation treated with polymer solution than in the preparation untreated with polymer solution.

**Table 4 In vivo sustained-release effect of hGH microparticle preparation**

| Polymer solution treatment | hGH content (%) | hGH concentration in blood (ng/mL) | | | | | | | |
|---|---|---|---|---|---|---|---|---|---|
| | | 4 hr | 8 hr | 1 day | 2 day | 3 day | 4 day | 5 day | 6 day |
| Untreated (n=2) | 16.2 | 2 6.4 | 4 9. 1 | 11.8 | 2.8 | 1.3 | 0.78 | 0.43 | 0.32 |
| Treated (n=2) | 11.2 | 42.6 | 65.4 | 21.6 | 9.7 | 4.4 | 2.2 | 1.4 | 0.49 |

### (Example 4)

HAp-Zn-0.5 (100 mg) treated at 400°C was supplemented with 700 µL of hGH solution (50 mg/mL) desalted with a PD-10 column (Amersham Pharmacia) and subsequently with water to bring the final amount of the solution to 5 mL. After stirring for 3 minutes, the solution was centrifuged at 3,000 rpm for 3 minutes. The resulting pellet was supplemented with 10 mL of water and stirred for 1 minute. The solution was centrifuged again at 3,000 rpm for 3 minutes. The resulting pellet was supplemented with 2.0 mL of aqueous solution of 20.4 mg/mL zinc chloride (300 µmol zinc chloride; Wako Pure Chemical Industries, Ltd., Osaka, Japan) and stirred with a touchmixer, followed by freeze drying.
PLA-PEG-PLA-Y004 (PEG ratio: 32%; molecular weight: 8,200) was dissolved at a concentration of 20% in acetone. This acetone solution was mixed with water at a 1:4 ratio to produce an acetone-water mixture solution containing the polymer. The resulting freeze-dried powder was supplemented with 500 µL of this acetone-water mixture solution containing the polymer and well stirred with a touch mixer, followed by freeze drying. A hGH content in the resulting hGH microparticle preparation was quantified with micro BCA protein assay kit (Pierce).

The produced hGH microparticle preparation was suspended in 0.5% CMC-Na, 5% mannitol, and 0.1% Tween 80 and subcutaneously administered at 30 IU/kg (1 IU: 0.35 mg) to the dorsal site of a male SD rat immunosuppressed with tacrolimus (Fujisawa Pharmaceutical Co., LTD., Osaka, Japan). The tacrolimus was administered in advance at a dose of 0.4 mg/rat on 3 days before the administration of the preparation and subsequently at a dose of 0.2 mg/rat at 3-day intervals after the initiation of subcutaneous administration of the preparation to the dorsal site.
Blood was collected from the tail vein at 1, 2, 4, and 8 hours after the administration of the preparation and subsequently every one or two days to measure a hGH concentration in blood with an automatic EIA apparatus AIA-6000 (Tosoh) and E Test "TOSOH" II (HGH). This result is shown in Table 5. The sustained-release effect over a period of approximately 2 weeks was observed in the hGH microparticle preparation treated with polymer solution.

Table 5 In vivo sustained-release effectofhGH microparticle preparation (hGH content: 14.3%)

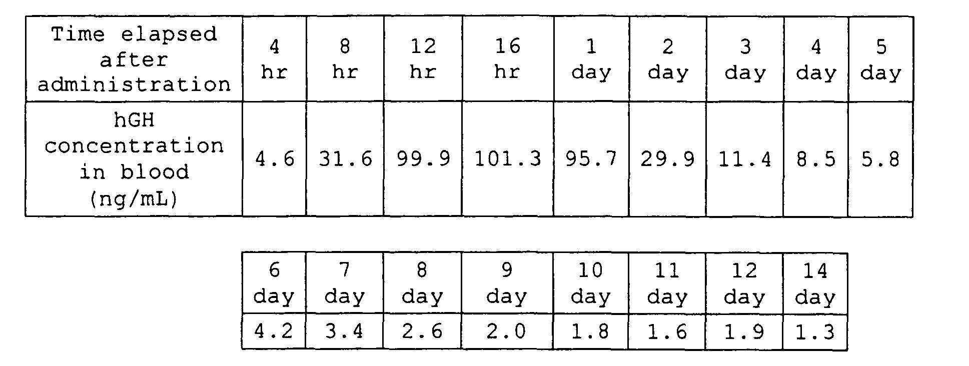

### (Example 5)

A derivative (HAp-Zn-0.5 (150 mg) : a portion of calcium in HAp was substituted with zinc; 0.5 mol zinc with respect to 9.5 mol calcium) burned at 400°C was supplemented with 525 µL of interferon-α (IFN-α) solution (2. 86 mg/mL) and then with water to bring the final amount of the solution to 2 mL. After stirring for 5 minutes, the suspension was centrifuged at 3, 000 rpm for 3 minutes. The resulting pellet was supplemented with 15 mL of water and stirred for 1 minute. The suspension was centrifuged again at 3, 000 rpm for 3 minutes. The resulting pellet was supplemented with 2.0 mL of aqueous solution of 20.4 mg/mL zinc chloride (300 µmol zinc chloride; Wako Pure Chemical Industries, Ltd., Osaka, Japan) and stirred with a touch mixer, followed by freeze drying. PLA-PEG-PLA (PEG ratio: 32%; molecular weight: 8,200) was dissolved at a concentration of 20% in acetone. This acetone solution was mixed with water at a 1:4 ratio to produce an acetone-water mixture solution containing the polymer. The resulting freeze-dried powder was supplemented with 750 µL of this acetone-water mixturesolution containing the polymer and well stirred with a touch mixer, followed by freeze drying. A preparation free of polymer solution treatment was produced as a control. An IFN-α content in the resulting HAp-IFN-α preparations was quantified with Human Interferon Alpha (Hu IFN-α) ELISA Kit (PBL Biomedical Laboratories).

The in vitro release capabilities of the resulting HAp-IFN-α samples were compared. The precisely weighed 2.5 mg aliquot of each of the resulting HAp-IFN-α samples was supplemented with 0.25 mL of 1/10 PBS (phosphate buffered saline) and stirred at 37°C. The supernatant was collected periodically by centrifugation at 3000 rpm for 3 minutes. The amount of IFN-α released into the supernatant was quantified with Human Interferon Alpha (Hu IFN-α) ELISA Kit (PBL Biomedical Laboratories). This result is shown in Table 6. The release of IFN-α into 1/10PBS was suppressed more sufficiently in the preparation treated with polymer solution than in the preparation untreated with polymer solution produced as a control.

**Table 6 Influence of polymer solution treatment on in vitro release capabilities of HAp-IFN-α preparation**

| | Cumulative amount of interferon-α released (pg) | | | | | | |
|---|---|---|---|---|---|---|---|
| | 0 | 1 hr | 2 hr | 4 hr | 24 hr | 4 day | 7 day |
| Untreated with polymer solution | 455 | 989 | 2601 | 3398 | 4017 | 4727 | 6338 |
| Treated with polymer solution | 95 | 207 | 328 | 410 | 550 | 641 | 752 |

### (Example 6)

HAp-Zn-0.5 (100 mg) treated at 400°C was supplemented with 3.5 mL of solution of a recombinant human insulin (Wako Pure Chemical Industries, Ltd., Osaka, Japan) dissolved at 10 mg/mL in 0.01 N HCl and further with 0.01 N HCl to bring the final amount of the solution to 5 mL. After stirring for 3 minutes, the suspension was centrifuged at 3,000 rpm for 3 minutes. The resulting pellet was supplemented with 10 mL of water and stirred for 1 minute. The suspension was centrifuged again at 3, 000 rpm for 3 minutes. The resulting pellet was freeze-dried. PLA-PEG-PLA-Y004 (PEG ratio: 32%; molecular weight: 8,200) was dissolved at a concentration of 20% in acetone. This acetone solution was mixed with water at a 1:4 ratio to produce an acetone-water mixture solution containing the polymer. The resulting freeze-dried powder was supplemented with 500 µL of this acetone-water mixture solution containing the polymer and well stirred with a touch mixer, followed by freeze drying. A preparation untreated with polymer solution was produced as a control. A hGH content in the resulting preparations was quantified with micro BCA protein assay kit (Pierce).

The in vitro release capabilities of the resulting insulin microparticle preparation samples were compared with the control. The precisely weighed 2. 5 mg aliquot of each of the resulting insulin microparticle preparation samples was supplemented with 0.25 mL of PBS (phosphate buffered saline) and stirred at 37°C. The supernatant was collected periodically by centrifugation at 3000 rpm for 3 minutes. The amount of insulin released into the supernatant was quantified with micro BCA protein assay kit (Pierce) to calculate its rate with respect to the total amount of insulin contained in each preparation. This result is shown in Table 7. The release of insulin into PBS was suppressed more in the preparation treated with polymer solution than in the preparation untreated with polymer solution produced as a control.

**Table 7 Influence of polymer solution treatment on in vitro release capabilities of insulin microparticle preparation**

| Polymer solution treatment | Cumulative amount of insulin released (% of total insulin) | | | | |
|---|---|---|---|---|---|
| | 1 hr | 2 hr | 4 hr | 24 hr | 4 day |
| Untreated (n=2) | 29.3 | 47.4 | 56.8 | 61.6 | 61.6 |
| Treated (n=2) | 21.8 | 39.8 | 49.0 | 49.0 | 49.0 |

## Claims

1. A protein drug sustained-release microparticle preparation for injection, **characterized by** comprising a porous apatite or derivative thereof containing a protein drug, coated with or adhered to, an in vivo disappearing polymer.

2. The protein drug sustained-release microparticle preparation for injection according to claim 1, **characterized in that** the in vivo disappearing polymer is a block copolymer consisting of polyethylene glycol and polylactic acid or copolylactic-glycolic acid.

3. The protein drug sustained-release microparticle preparation for injection according to claim 2, **characterized in that** the block copolymer consisting of polyethylene glycol and polylactic acid or copolylactic-glycolic acid is a block copolymer consisting of polylactic acid or copolylactic-glycolic acid-polyethylene glycol-polylactic acid or copolylactic-glycolic-acid.

4. The protein drug sustained-release microparticle preparation for injection according to claim 2, **characterized in that** the block copolymer consisting of polyethylene glycol and polylactic acid or copolylactic-glycolic acid has a weight-average molecular weight of 3,000 to 20,000.

5. The protein drug sustained-release microparticle preparation for injection according to claim 2 or 3, **characterized in that** the block copolymer consisting of polyethylene glycol and polylactic acid or copolylactic-glycolic acid has 20 to 90% by weight of polyethylene glycol.

6. The protein drug sustained-release microparticle preparation for injection according to claim 1, **characterized in that** the porous apatite or derivative thereof contains a protein drug and a divalent metal salt.

7. The protein drug sustained-release microparticle preparation for injection according to claim 1, **characterized in that** the porous apatite or derivative thereof has a protein drug content of 5 to 30%.

8. The protein drug sustained-release microparticle preparation for injection according to claim 1, **characterized in that** the porous apatite or derivative thereof has an average particle size of 0.5 to 30 µm.

9. The protein drug sustained-release microparticle preparation for injection according to claim 1, **characterized in that** the porous apatite or derivative thereof is treated in the range from 100 to 600°C.

10. The protein drug sustained-release microparticle preparation for injection according to claim 1, **characterized in that** the porous apatite or derivative thereof is an apatite derivative in which a portion of calcium in the porous apatite is substituted with zinc.

11. A process for producing a protein drug sustained-release microparticle preparation for injection, **characterized by** comprising dispersing microparticles of a porous apatite or derivative thereof in an aqueous solution of a protein drug, stirring the dispersion, dispersing the resulting powder in an aqueous solution or suspension of a biodegradable polymer, stirring the dispersion, and then freeze drying or vacuum drying to give a powder.
